# EUROPEAN PATENT SPECIFICATION

(11) **EP 2 498 088 B1**
(45) Date of publication and mention of the grant of the patent: **06.05.2015**
(21) Application number: 12157958.5
(22) Date of filing: 02.03.2012
(51) Int. Cl.: G01N 27/60, G01N 15/06, G01N 33/34, G01N 27/26

(54) **The determination of an electrical characteristic of a particles containing liquid**
Bestimmung eines elektrischen Merkmals von flüssigkeitshaltigen Partikeln
Détermination d'une caractéristique électrique d'un liquide contenant des particules

(30) Priority: 09.03.2011 NL 2006363
(43) Date of publication of application: 12.09.2012
(73) Proprietor: Adviesburo Magendans B.V., 6673 MA Andelst (NL)
(72) Inventor: Magendans, Nico, 6673 MA Andelst (NL); van der Meyden, Wim, 6673 MA ANDELST (NL)
(74) Representative: EP&C

(56) References cited:
- EP-A1- 0 462 703
- EP-A1- 0 869 357
- WO-A2-2007/045481
- WO-A2-2007/057160

## Description

The present invention relates to the determination of an electrical characteristic, in particular the streaming potential or zeta-potential, of a particles containing liquid.

In the art of making paper it is generally known to determine the zeta-potential of the pulp in order to control the papermaking process. Other well-known applications of zeta-potential determination are found in the treatment of sewage water and other effluents.

Determination of the zeta-potential is also known for suspensions, fibers containing liquids, colloids, (human) blood, etc.

WO2007045481 discloses an example of a system for the determination of the zeta-potential.

It is an object of the present invention to provide an improved system.

The invention provides a system according to the preamble of claim 1, which is **characterized in that** the system further comprises:
- an intermittent filtering web feed mechanism, and
- a roll of disposable filtering web or a zigzag folded continuous disposable filtering web, wherein the measurement apparatus has a path for the disposable filtering web that extends across the measuring conduit so that a portion of the web in said measuring conduit forms the particles retaining filter,
and wherein the intermittent filtering web feed mechanism is adapted to advance said filtering web intermittently so that as to replace a portion of filtering web used as particles retaining filter in a measurement cycle for a clean and non-used portion of the filtering web for the next measurement cycle.

The system according to the present invention can be used for liquids having a very low particles content and/or with very small particles, as the plug is established using the filtering web. The web is appropriately chosen for the liquid to be handled.

An advantage of the system according to the invention is that the plug is established on the filtering web, and discarded along with the filtering web. This avoids a problem of known systems with a sieve type electrode to establish the plug, wherein said sieve type electrode is soiled by the material forming the plug and in practice requires extensive cleaning before it is suitable for a further measurement cycle.

The system according to the present invention is primarily envisaged as a batch system, wherein a batch of liquid to be examined is introduced into the measurement apparatus. In another embodiment the system can be used as an "in-line determination system", wherein a (sample) flow of liquid is fed to the measurement apparatus, e.g. periodically.

In a preferred embodiment one of the electrodes comprises one or more members extending across the measuring conduit, e.g. the electrode being embodied as a metallic sieve, and the path for the filtering web extends along said electrode such that in use the filtering web is supported by the one or more members of said electrode. This allows for the use of fragile filtering web material, avoids tearing of the filtering web during the measurement cycle, and provides for a stable position of the plug of particles during the cycle.

As is preferred the one or more members of said filtering web supporting electrode support the filtering web at the side thereof opposite from the side where the plug of particles is established. Thereby the filtering web avoids soiling of said electrode.

In an embodiment wherein one electrode is a sieve type electrode, the other electrode can e.g. be embodied as an annular electrode having a central opening for the passage of particles containing liquid, e.g. received in the wall of the measurement conduit.

In a practical embodiment the filtering web is a filtering paper web.

In a practical embodiment the system includes a roll of disposable filtering web, and the web feed mechanism includes a source roller assembly adapted to unroll the roll of filtering web and a take-up roller assembly adapted to roll up the used filtering web.

In a preferred embodiment, the controllable variable liquid flow means comprise a vacuum device that is in fluid communication with the measuring conduit and is adapted to establish a pressure difference variation across the plug of articles during the measurement cycle.

In a practical embodiment the vacuum device comprises a piston and cylinder device having a cylinder and a reciprocable piston therein delimiting a variable volume chamber in fluid communication with said measuring conduit with an associated drive motor for effecting a controlled displacement of said piston with respect to said cylinder.

In a preferred embodiment the vacuum device is adapted to cause a predetermined periodic variation of the pressure in said variable volume chamber.

In a practical embodiment of a batch system, the measurement apparatus includes a liquid container, e.g. an open topped liquid container, which can contain a batch of liquid to be analyzed, said container being in fluid communication with said measurement conduit. In a practical embodiment the measurement conduit connects to the bottom of the container and extends downward, e.g. vertically, from said bottom.

In a practical embodiment the measuring conduit is arranged in vertical orientation within a housing, said first and second electrode being arranged above one another.

In a practical embodiment the system includes a pressure sensor that allows to measure the pressure in a variable volume chamber that is in communication with the measurement conduit, and a control means associated with the drive motor that effects variation of the volume of the variable volume chamber, said control means being adapted to establish a predetermined pattern of variation of the pressure in said variable volume chamber.

In a practical embodiment the drive motor is a linear actuator, e.g. a linear motor or a pneumatic or hydraulic cylinder, preferably including a position sensor to detect a piston position and/or speed, e.g. with a servo control loop, e.g. a linear motor or a pneumatic or hydraulic cylinder. In another embodiment the drive motor has a rotary output shaft, connected to the piston via a crankshaft - connecting rod mechanism.

In a preferred embodiment the system further comprises a pressure sensor adapted to measure the pressure difference across the plug of particles, said pressure sensor assembly being interconnected to said signal processing means.

In an embodiment the system comprises a conductivity sensor adapted to measure the conductivity of the liquid, e.g. whilst in said measurement conduit, e.g. the first and second electrodes being part of the conductivity sensor, said conductivity sensor being interconnected to said signal processing means.

The invention also relates to a method for the determination of an electrical characteristic, in particular the streaming potential or zeta-potential, of a particles containing liquid, said method including the use of a system according to the invention.

The invention will now be described in more detail with reference to the appended drawings.

In the drawings:
fig. 1 shows schematically an example of a system according to the invention,
fig. 2 shows schematically a portion of the system of figure 1 in perspective view.

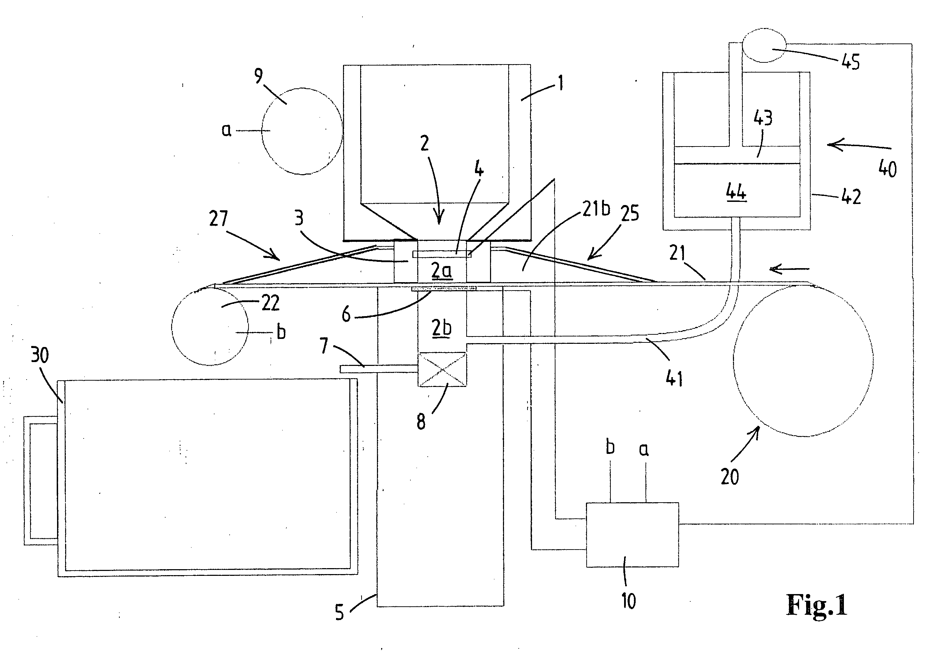

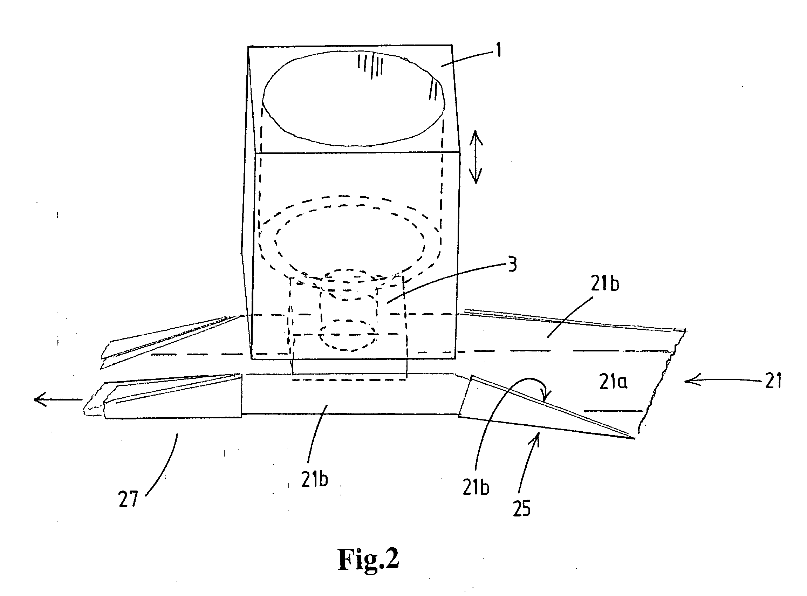

With reference to figures 1 and 2 now an exemplary embodiment, and preferred and optional details, of a system for the determination of an electrical characteristic, in particular the streaming potential or zeta-potential, of a particles containing liquid in a measurement cycle will be explained.

In general the system shown here is designed for measurements to be performed on batches of particles containing liquid, e.g. for use in a laboratory.

The system includes a measurement apparatus having a liquid container 1, here, is preferred, an open topped liquid container 1, which can contain a batch of liquid to be analyzed.

The container 1 is in fluid communication with a measurement conduit 2. Here, as is preferred, the container 1 is integral with a liftable measuring conduit member 3, that forms a portion, here an upper part 2a, of the measuring conduit. The member 3, in this example along with the container 1, is movable between an operative and a retracted position, here a lowered and a raised position. The operation will be explained in more detail below.

As is preferred the conduit member 3 includes a first electrode 4 which contacts the liquid in the measuring conduit during the measurement cycle, as is preferred the electrode 4 is a ring electrode, e.g. having an inner diameter the same as the diameter of the conduit 2.

The apparatus here further comprises a housing 5 in which a further, here lower, part 2b of the measuring conduit 2 is present.

The housing 5 supports a second electrode 6, spaced from the first electrode 4, here in vertical direction.

As is preferred the second electrode 6 comprises one or more members extending across the measuring conduit, here the electrode 6 is embodied as a sieve, e.g. a metallic sieve.

A signal processing and control means 10 is connected to the first and second electrodes 4, 6 for the determination of the electrical characteristic of the liquid in a measurement cycle, such means 10 are known in the art.

In this example, as is preferred, the measuring conduit 2 is arranged in vertical orientation, said first and second electrodes 4, 6 being arranged above one another.

The system further includes an intermittent filtering web feed mechanism for feeding a filtering web 21 to the position across the measuring conduit 2.

The filtering web here is provided in the form of a roll 20 of disposable filtering web 21, as is preferred. In an alternative a zigzag folded continuous disposable filtering web is provided. The apparatus preferably comprises a source roller assembly (not shown here, e.g. a supporting spindle) adapted to unroll or allow for the unrolling of the roll 20 of filtering web 21.

As is preferred the web 21 is a paper filtering web. It is noted that filtering webs are commercially available with well defined filtering properties. A suitable web can be chosen in combination with the liquid to be examined, possibly some comparative tests can be done to select an optimal filtering web.

It is noted that filtering webs are usually very thin, and the thickness has been exaggerated in the figures for reasons of clarity.

The measurement apparatus has a path for the disposable filtering web that extends across the measuring conduit, so that a portion of the web 21 in said measuring conduit forms the particles retaining filter. As will be explained a plug of particles will form on said web during the measurement cycle.

In this example, as is preferred, the mechanism includes a driven take-up roller 22, e.g. with an electric drive motor, that is operated such that the web is intermittently advanced from the supply roll 20 to the take-up roller 22. It will be appreciated that said intermittent advancing can be realized with differently embodied motorized intermittent advancing means.

The intermittent filtering web feed mechanism is adapted to advance the filtering web intermittently so that as to replace the portion of filtering web 21 used as particles retaining filter in a measurement cycle for a clean and non-used portion of the filtering web for the next measurement cycle.

As is preferred the path for the filtering web 21 extends along the second electrode 6, here - as is preferred - above the electrode, such that in use the filtering web 21 (and the plug formed thereon) is supported by the one or more members of said electrode 6. As is preferred the members of the second electrode 6 support the filtering web 21 at the side thereof opposite from the side where the plug is established.

In the operative position - the liftable measuring conduit member 3 sealingly engages on the filtering web 21 at the side where the plug of particles is formed during the measurement cycle. In practice the filtering web 21 is then suitably clamped between said member 3 and the housing 5.

In the retracted, here raised, position, the liftable measuring conduit member 3 is spaced from the filtering web 21 so as to allow for the used portion of the filtering web 21 with the plug of particles thereon to be advanced in a discharge direction.

As can be seen a lifting means 9 is associated with the liftable member 3, here also with the container 1, to perform the motion of the member 3 when desired. As is preferred the lifting means 9 is linked to the control means 10, here via control line a, to obtain an automated operation of the system.

As is preferred the advancing means, here take-up roller drive, is linked to the means 10, here via control line b, to obtain an automated operation of the system.

As is preferred the system includes a waste container, e.g. a portable container 30, in which the liquid is received after performing a measurement cycle, here via discharge conduit 7 that is selectively connectable to lower conduit part 2b via the (electronic controlled) valve 8.

As is further preferred, the web feed mechanism is adapted to advance the web 21 with the plug thereon so that said plug falls into a waste collection container as well, preferably both the plug and the liquid being received in a common waste container 30.

To perform a measurement cycle the apparatus further comprises a controllable variable liquid flow means that is adapted to generate during a measurement cycle a controlled liquid flow variation across the plug of particles on said filtering web 21 for the purpose of determination of said electrical characteristic of said liquid with the electrodes and associated signal processing means.

As is preferred the controllable variable liquid flow means here comprise a vacuum device 40 that is, here via conduit 41, in fluid communication with the measuring conduit 2, here the portion 2b thereof in the housing 5. The vacuum device is adapted to establish a pressure difference variation across the plug of articles retained on the web 21 during the measurement cycle.

In this example, as is preferred, the vacuum device 40 is not a reciprocating or rotary vacuum pump, but is a piston and cylinder device having a cylinder 42 and a piston 43 therein delimiting a variable volume chamber 44 in fluid communication with said measuring conduit 2. The associated drive motor 45 effects a controlled motion of said piston with respect to said cylinder, such that the position of the piston 43 corresponds to a desired vacuum which translates into a desired pressure difference across the plug of particles and the filtering web. So the piston is not reciprocated several times to obtain the desired vacuum as in a reciprocating piston pump having a small stroke volume. The maximum volume of the chamber 44 is such that the maximum desired vacuum can be established by moving the piston from one end position to the other end position.

The drive motor 45 may include a linear actuator, e.g. a linear motor or a pneumatic or hydraulic cylinder.

Preferably a position sensor is present to detect the piston position and/or speed, e.g. with a servo control loop to the associated drive motor.

As can be seen the drive motor 45 is linked to the means 10 to control the operation of the vacuum device 40.

The system may preferably include a pressure sensor that allows to measure the pressure in the variable volume chamber, and a control means associated with the drive motor 45, said control means being adapted to establish a predetermined pattern of variation of the pressure in said variable volume chamber, e.g. by effecting that the piston 43 moves back and fro several times thereby causing a periodic variation of the vacuum over time between minimum and maximum values during the measurement cycle.

For example the vacuum device is adapted to cause a predetermined periodic variation of the pressure in the variable volume chamber 44.

The system may comprises a pressure sensor that is adapted to measure the pressure difference for the liquid across the plug of particles, said pressure sensor assembly being interconnected to the signal processing and control means 10, e.g. in view of the calculation of the electrical characteristic of the liquid by said means.

The system may comprise a conductivity sensor, e.g. making use of the aforementioned electrodes, that is adapted to measure the conductivity of the liquid, said conductivity sensor being interconnected to said signal processing means, e.g. in view of the calculation of the electrical characteristic of the liquid by said means.

In a preferred embodiment, as illustrated here, the web feed mechanism comprises a filtering web guide and shaping structure 25 that is arranged to engage on the filtering web 21 before it reaches the measuring conduit, and that is adapted to shape, here fold, the web into a U-shaped cross-section with a central web portion 21 a upon which the plug of particles is retained during the measurement cycle, and with raised side web portions 21 b.

At the location of the measurement conduit, here at member 3, the web 21 is in said U-shape, with the side web portions 21 b here passing on opposed sides of the member 3 (or extending through open-bottom channels in said member 3).

The web feed mechanism in this example also comprises a guide structure 27 that is arranged to engage on the filtering web downstream of the measuring conduit, and that is adapted to maintain a U-shaped cross-section of the web thereby allowing the web 21 to act as a discharge gutter for the particles plug and possibly for (the remainder of) a batch of liquid that was present in a liquid container of the measurement apparatus.

In this example it is shown that the guide structure 27 is embodied to bring the edges of the web 21 gradually close together, so as to place the side web portions 21 b on top of the central web portion 21a. This facilitates the winding up of the soiled web.

As can be seen the plug of particles on the web is allowed to be discharged into the container 30.

The filtering web 21 allows for the desired built-up of a plug of particles, e.g. the filtering property being chosen in view of the expected size of the particles in the liquid or other particle/liquid properties.

The plug does not soil the electrode 6 underneath the filtering web, thereby avoid cleaning thereof or at least facilitating the cleaning of said electrode (and the lower part 2b of the conduit).

If desired the system could include a cleaning system for cleaning parts of the device, e.g. to establish a cleaning liquid flow along portions of interest.

The path shown in figure 1 for the web 21 is shown as horizontal. It is envisaged that in another embodiment said path is slanting down in discharge direction of the web, e.g. so as to avoid that (a remainder of) liquid from the container streams towards the supply roll 20. A barrier to counteract said effect may be provided as well if desired.

## Claims

1. A system for the determination of an electrical characteristic, in particular the streaming potential or zeta-potential, of a particles containing liquid in a measurement cycle, said system including:
- a measurement apparatus which comprises:
- a measuring conduit (2, 2a, 2b) for said liquid,
- a first electrode (4) and a second electrode (6) spaced from each other along the measuring conduit (2), so that each electrode (4, 6) contacts said liquid in said measuring conduit during the measurement cycle,
- a signal processing means (10) connected to said first and second electrodes (4, 6) for the determination of the electrical characteristic of said liquid in said measurement cycle,
- a particles retaining filter effective at a location in said measuring conduit during the measurement cycle, which particles retaining filter allows for the build up and retention of a plug of particles for said measurement cycle,
- a controllable variable liquid flow means (40, 41)adapted to generate during said measurement cycle a controlled liquid flow variation across the plug of particles on said filter for the purpose of determination of said electrical characteristic of said liquid,
**characterized in that** the system further comprises:
- an intermittent filtering web feed mechanism (22, 25, 27), and
- a roll of disposable filtering web (20) or a zigzag folded continuous disposable filtering web, wherein the measurement apparatus has a path for the disposable filtering web that extends across the measuring conduit (2) so that a portion of the web in said measuring conduit forms the particles retaining filter,
and wherein the intermittent filtering web feed mechanism is adapted to advance said filtering web (21) intermittently so that as to replace the portion of filtering web used as particles retaining filter in a measurement cycle for a clean and non-used portion of the filtering web for the next measurement cycle.

2. System according to claim 1, wherein one of the electrodes (6) comprises one or more members extending across the measuring conduit, e.g. the electrode being embodied as a metallic sieve (6), and wherein the path for the filtering web (21) extends along said electrode such that in use the filtering web is supported by the one or more members of said electrode, and wherein, preferably, said members support the filtering web (21) at the side thereof opposite from the side where the plug is established.

3. System according to one or more of the preceding claims, wherein the filtering web (21) is a filtering paper web.

4. System according to one or more of the preceding claims, wherein the system includes a roll (20) of disposable filtering web, and wherein the web feed mechanism includes a source roller assembly adapted to unroll the roll of filtering web and a take-up roller assembly (22) adapted to roll up the used filtering web.

5. System according to one or more of the preceding claims, wherein the web feed mechanism comprises a filtering web guide and shaping structure (25) that is arranged to engage on the filtering web (21) as it advances towards the measuring conduit (2), and that is adapted to shape the web into a U-shaped cross-section with a central web portion (21 a) upon which the plug of particles is retained during the measurement cycle, and with raised side web portions (21b), said web feed mechanism also comprising a guide structure (27) that is arranged to engage on the filtering web (21) downstream of the measuring conduit, and that is adapted to maintain a U-shaped cross-section of the web thereby allowing the web to act as a discharge gutter for the particles plug and possibly for a batch of liquid that was present in a liquid container of the measurement apparatus.

6. System according to one or more of the preceding claims, wherein said measurement apparatus includes a liquid container (1), e.g. an open topped liquid container, which can contain a batch of liquid to be analyzed, said container being in fluid communication with said measurement conduit (2).

7. System according to one or more of the preceding claims, preferably according to claim 5, wherein the measurement apparatus comprises a liftable measuring conduit member (3) that is movable between an operative and a retracted position, wherein - in the operative position - said liftable measuring conduit member sealingly engages on the filtering web (21) at the side where the plug of particles is formed during the measurement cycle, and wherein - in the retracted position - the liftable measuring conduit member (3) is spaced from the filtering web so as to allow for the plug to be discharged with the filtering web, and wherein, preferably, an electrode is arranged in said liftable measuring conduit member.

8. System according to claims 6 and 7, wherein said liquid container (1) is integral with said liftable measuring conduit member (3).

9. System according to one or more of the preceding claims, wherein the controllable variable liquid flow means comprise a vacuum device (40) that is in fluid communication with the measuring conduit (2) and is adapted to establish a pressure difference variation across the plug of articles during the measurement cycle.

10. System according to claim 9, wherein the vacuum device comprises a piston and cylinder device having a cylinder (42) and a reciprocable piston (43) therein delimiting a variable volume chamber (44) in fluid communication with said measuring conduit (2), wherein an associated drive motor (45) is provided for effecting a controlled displacement of said piston with respect to said cylinder, and wherein, preferably, the vacuum device is adapted to cause a predetermined periodic variation of the pressure in said variable volume chamber (44).

11. System according to one or more of the preceding claims, wherein said measuring conduit (2) is arranged in vertical orientation, said first and second electrodes (4,6) being arranged above one another.

12. System according to claim 10, further including a pressure sensor that allows to measure the pressure in said variable volume chamber (44), and a control means (10) associated with the drive means (45), said control means being adapted to establish a predetermined pattern of variation of the pressure in said variable volume chamber (44).

13. System according to one or more of the preceding of the claims, wherein said system further comprises a pressure sensor adapted to measure the pressure difference across the plug of particles, said pressure sensor being interconnected to said signal processing means.

14. System according to one or more of the preceding of claims, wherein the system comprises a conductivity sensor adapted to measure the conductivity of the liquid, said conductivity sensor being interconnected to said signal processing means.

15. Method for the determination of an electrical characteristic, in particular the streaming potential or zeta-potential, of a liquid, said method including the use of a system according to one or more of the preceding claims.

## Patentansprüche

1. System für die Bestimmung einer elektrischen Eigenschaft, insbesondere des Strömungspotenzials oder Zeta-Potenzials, einer Partikel enthaltenden Flüssigkeit in einem Messzyklus, wobei das System aufweist:
- eine Messvorrichtung, welche umfasst:
- eine Messleitung (2, 2a, 2b) für die Flüssigkeit,
- eine erste Elektrode (4) und eine zweite Elektrode (6), welche voneinander entlang der Messleitung (2) beabstandet sind, so dass jede Elektrode (4, 6) die Flüssigkeit in der Messleitung während des Messzyklus berührt,
- Signalverarbeitungsmittel (10), welche für die Bestimmung der elektrischen Eigenschaft der Flüssigkeit in dem Messzyklus mit der ersten und der zweiten Elektrode (4, 6) verbunden sind,
- ein Partikel zurückhaltendes Filter, welches sich effektiv an einer Stelle in der Messleitung während des Messzyklus befindet, wobei das Partikel zurückhaltende Filter den Aufbau und das Zurückhalten eines Pfropfens von Partikeln für den Messzyklus ermöglicht,
- steuerbare variable Flüssigkeitsstrommittel (40, 41), welche ausgestaltet sind, um während des Messzyklus eine gesteuerte Flüssigkeitsstromänderung über dem Pfropfen von Partikeln auf dem Filter zum Zweck einer Bestimmung der elektrischen Eigenschaft der Flüssigkeit zu erzeugen,
**dadurch gekennzeichnet, dass** das System darüber hinaus umfasst:
- einen Mechanismus (22, 25, 27) für eine unterbrochene Zuführung eines filternden Materials, und
- eine Rolle eines wegwerfbaren filternden Materials (20) oder eines zickzackförmig gefalteten kontinuierlichen wegwerfbaren filternden Materials, wobei die Messvorrichtung eine Bahn für das wegwerfbare filternde Material aufweist, welche sich über die Messleitung (2) erstreckt, so dass ein Abschnitt des Materials in der Messleitung das Partikel zurückhaltende Filter ausbildet,
und wobei der Mechanismus für die unterbrochene Zuführung des filternden Materials ausgestaltet ist, um das filternde Material (21) mit Unterbrechungen vorwärts zu bewegen, um so den Abschnitt des filternden Materials, welcher als das Partikel zurückhaltende Filter in einem Messzyklus eingesetzt wird, durch einen sauberen und nicht verwendeten Abschnitt des filternden Materials für den nächsten Messzyklus zu ersetzen.

2. System nach Anspruch 1, wobei eine der Elektroden (6) ein oder mehrere Teile umfasst, welche sich über die Messleitung erstrecken, wobei die Elektrode z.B. als ein Metallsieb (6) ausgestaltet ist, und wobei sich die Bahn für das filternde Material (21) entlang der Elektrode erstreckt, so dass das filternde Material im Betrieb durch das eine oder die mehreren Teile der Elektrode gehalten wird, und wobei vorzugsweise die Teile das filternde Material (21) an der Seite davon halten, welche der Seite gegenüberliegt, wo der Pfropfen aufgebaut ist.

3. System nach einem oder mehreren der vorhergehenden Ansprüche, wobei das filternde Material (21) ein filterndes Papiermaterial ist.

4. System nach einem oder mehreren der vorhergehenden Ansprüche, wobei das System eine Rolle (20) eines wegwerfbaren filternden Materials aufweist, und wobei der Mechanismus für die Zuführung des Materials eine Ausgangsrollenanordnung, welche ausgestaltet ist, um die Rolle des filternden Materials abzurollen, und eine aufnehmende Rollenanordnung (22), welche ausgestaltet ist, um das verwendete filternde Material aufzurollen, aufweist.

5. System nach einem oder mehreren der vorhergehenden Ansprüche, wobei der Mechanismus für die Zuführung des Materials eine Führungs- und Formungsstruktur (25) für das filternde Material umfasst, welche ausgestaltet ist, um auf dem filternden Material (21) in Eingriff zu kommen, wenn es sich zu der Messleitung (2) vor bewegt, und welche ausgestaltet ist, um das Material zu formen in einen U-förmigen Querschnitt mit einem zentralen Materialabschnitt (21 a), auf welchem der Pfropfen von Partikeln während des Messzyklus zurückgehalten wird, und mit erhöhten seitlichen Materialabschnitten (21 b), wobei der Mechanismus für die Zuführung des Materials auch eine Führungsstruktur (27) umfasst, welche ausgestaltet ist, um stromabwärts der Messleitung auf dem filternden Material (21) in Eingriff zu kommen, und welche ausgestaltet ist, um einen U-förmigen Querschnitt des Materials zu erhalten, um dadurch dem Material zu ermöglichen, als eine Ablassrinne für den Partikel-Pfropfen und möglicherweise für eine Ladung einer Flüssigkeit, welche in einem Flüssigkeitsbehälter der Messvorrichtung vorhanden war, zu wirken.

6. System nach einem oder mehreren der vorhergehenden Ansprüche, wobei die Messvorrichtung einen Flüssigkeitsbehälter (1), z.B. einen oben offenen Flüssigkeitsbehälter, welcher eine Ladung einer zu analysierenden Flüssigkeit enthalten kann, aufweist, wobei sich der Behälter in einer Fluidverbindung mit der Messleitung (2) befindet.

7. System nach einem oder mehreren der vorhergehenden Ansprüche, vorzugsweise nach Anspruch 5, wobei die Messvorrichtung ein anhebbares Messleitungsteil (3) umfasst, welches zwischen einer betriebsfähigen und einer eingezogenen Position bewegbar ist, wobei sich das anhebbare Messleitungsteil - in der betriebsfähigen Position - abdichtend an der Seite auf dem filternden Material (21) in Eingriff befindet, auf welcher der Pfropfen von Partikeln während des Messzyklus ausgebildet wird, und wobei das anhebbare Messleitungsteil (3) - in der eingezogenen Position - von dem filternden Material beabstandet ist, um so zu ermöglichen, dass der Pfropfen mit dem filternden Material abgenommen wird, und wobei vorzugsweise eine Elektrode in dem anhebbaren Messleitungsteil angeordnet ist.

8. System nach den Ansprüchen 6 und 7, wobei der Flüssigkeitsbehälter (1) integral mit dem anhebbaren Messleitungsteil (3) ausgebildet ist.

9. System nach einem oder mehreren der vorhergehenden Ansprüche, wobei die steuerbaren variablen Flüssigkeitsstrommittel eine Vakuumvorrichtung (40) umfassen, welche sich in einer Fluidverbindung mit der Messleitung (2) befindet und ausgestaltet ist, um eine Druckdifferenzänderung über dem Pfropfen von Partikeln während des Messzyklus aufzubauen.

10. System nach Anspruch 9, wobei die Vakuumvorrichtung eine Kolben- und Zylindervorrichtung mit einem Zylinder (42) und einem hin und her bewegbaren Kolben (43) darin umfasst, welche eine variable Volumenkammer (44) in Fluidverbindung mit der Messleitung (2) abgrenzt, wobei ein zugeordneter Antriebsmotor (45) vorhanden ist, um eine gesteuerte Verschiebung des Kolbens bezüglich des Zylinders zu bewirken, und wobei vorzugsweise die Vakuumvorrichtung ausgestaltet ist, um eine vorbestimmte periodische Änderung des Drucks in der variablen Volumenkammer (44) zu bewirken.

11. System nach einem oder mehreren der vorhergehenden Ansprüche, wobei die Messleitung (2) in vertikaler Orientierung angeordnet ist, wobei die erste und die zweite Elektrode (4, 6) übereinander angeordnet sind.

12. System nach Anspruch 10, darüber hinaus aufweisend einen Drucksensor, welcher ermöglicht, den Druck in der variablen Volumenkammer (44) zu messen, und Steuermittel (10), welche den Antriebsmitteln (45) zugeordnet sind, wobei die Steuermittel ausgestaltet sind, um ein vorbestimmtes Muster einer Änderung des Drucks in der variablen Volumenkammer (44) herzustellen.

13. System nach einem oder mehreren der vorhergehenden Ansprüche, wobei das System darüber hinaus einen Drucksensor umfasst, welcher ausgestaltet ist, um die Druckdifferenz über dem Pfropfen von Partikeln zu messen, wobei der Drucksensor mit den Signalverarbeitungsmitteln verbunden ist.

14. System nach einem oder mehreren der vorhergehenden Ansprüche, wobei das System einen Leitfähigkeitssensor umfasst, welcher ausgestaltet ist, um die Leitfähigkeit der Flüssigkeit zu messen, wobei der Leitfähigkeitssensor mit den Signalverarbeitungsmitteln verbunden ist.

15. Verfahren für die Bestimmung einer elektrischen Eigenschaft, insbesondere des Strömungspotenzials oder Zeta-Potenzials, einer Flüssigkeit, wobei das Verfahren die Verwendung eines Systems nach einem oder mehreren der vorhergehenden Ansprüche aufweist.

## Revendications

1. Système pour la détermination d'une caractéristique électrique, en particulier du potentiel électrocinétique ou du potentiel zêta, d'un liquide contenant des particules dans un cycle de mesure, ledit système comprenant :
- un appareil de mesure qui comprend :
- un conduit de mesure (2, 2a, 2b) pour ledit liquide,
- une première électrode (4) et une deuxième électrode (6) espacées l'une de l'autre le long du conduit de mesure (2), de sorte que chaque électrode (4, 6) soit en contact avec ledit liquide dans ledit conduit de mesure pendant le cycle de mesure,
- des moyens de traitement de signal (10) connectés aux dites première et deuxième électrodes (4, 6) pour la détermination de la caractéristique électrique dudit liquide dans ledit cycle de mesure,
- un filtre de retenue de particules efficace à un emplacement dans ledit conduit de mesure pendant le cycle de mesure, lequel filtre de retenue de particules permet le développement et la retenue d'un bouchon de particules pendant ledit cycle de mesure,
- des moyens d'écoulement de liquide variable pouvant être contrôlé (40, 41) conçus pour générer, pendant ledit cycle de mesure, une variation d'écoulement de liquide contrôlée de part et d'autre du bouchon de particules sur ledit filtre afin de déterminer ladite caractéristique électrique dudit liquide,
**caractérisé en ce que** le système comprend en outre :
- un mécanisme d'avance intermittente de bande de filtration (22, 25, 27), et
- un rouleau de bande de filtration jetable (20) ou une bande de filtration jetable continue pliée en zigzag, dans lequel l'appareil de mesure a un trajet pour la bande de filtration jetable qui s'étend à travers le conduit de mesure (2) de sorte qu'une partie de la bande dans ledit conduit de mesure forme le filtre de retenue de particules,
et dans lequel le mécanisme d'avance intermittente de bande de filtration est conçu pour avancer ladite bande de filtration (21) par intermittence de manière à remplacer la partie de bande de filtration utilisée en tant que filtre de retenue de particules dans un cycle de mesure par une partie propre et non utilisée de la bande de filtration pour le cycle de mesure suivant.

2. Système selon la revendication 1, dans lequel l'une des électrodes (6) comprend un ou plusieurs éléments s'étendant à travers le conduit de mesure, par exemple l'électrode mise en oeuvre en tant que tamis métallique (6), dans lequel le trajet pour la bande de filtration (21) s'étend le long de ladite électrode de sorte que, en utilisation, la bande de filtration soit supportée par lesdits un ou plusieurs éléments de ladite électrode, et dans lequel, de préférence, lesdits éléments supportent la bande de filtration (21) d'un côté de celle-ci opposé au côté où le bouchon est établi.

3. Système selon une ou plusieurs des revendications précédentes, dans lequel la bande de filtration (21) est une bande de papier de filtration.

4. Système selon une ou plusieurs des revendications précédentes, dans lequel le système comprend un rouleau (20) de bande de filtration jetable, et dans lequel le mécanisme d'avance de bande comprend un ensemble de rouleau de source conçu pour dérouler le rouleau de bande de filtration et un ensemble de rouleau d'enroulement (22) conçu pour enrouler la bande de filtration utilisée.

5. Système selon une ou plusieurs des revendications précédentes, dans lequel le mécanisme d'avance de bande comprend une structure de guidage et de mise en forme de bande de filtration (25) qui est agencée pour venir en prise avec la bande de filtration (21) alors qu'elle avance vers le conduit de mesure (2), et qui est conçue pour mettre la bande en forme de U en coupe avec une partie de bande centrale (21a) sur laquelle le bouchon de particules est retenu pendant le cycle de mesure, et avec des parties de bande latérales relevées (21b), ledit mécanisme d'avance de bande comprenant également une structure de guidage (27) qui est agencée pour venir en prise avec la bande de filtration (21) en aval du conduit de mesure, et qui est conçue pour maintenir une forme en U en coupe de la bande, permettant de ce fait à la bande d'agir en tant que gouttière de déchargement pour le bouchon de particules et éventuellement pour un lot de liquide qui était présent dans un récipient de liquide de l'appareil de mesure.

6. Système selon une ou plusieurs des revendications précédentes, dans lequel ledit appareil de mesure comprend un récipient de liquide (1), par exemple un récipient de liquide ouvert sur le dessus, qui peut contenir un lot de liquide à analyser, ledit récipient étant en communication fluidique avec ledit conduit de mesure (2).

7. Système selon une ou plusieurs des revendications précédentes, de préférence selon la revendication 5, dans lequel l'appareil de mesure comprend un élément de conduit de mesure levable (3) qui peut être déplacé entre une position de fonctionnement et une position rétractée, dans lequel - dans la position de fonctionnement - ledit élément de conduit de mesure levable vient en prise de manière étanche avec la bande de filtration (21) du côté où le bouchon de particules est formé pendant le cycle de mesure, et dans lequel - dans la position rétractée - l'élément de conduit de mesure levable (3) est espacé de la bande de filtration de manière à permettre le déchargement du bouchon avec la bande de filtration, et dans lequel, de préférence, une électrode est agencée dans ledit élément de conduit de mesure levable.

8. Système selon les revendications 6 et 7, dans lequel ledit récipient de liquide (1) est d'un seul tenant avec ledit élément de conduit de mesure levable (3).

9. Système selon une ou plusieurs des revendications précédentes, dans lequel les moyens d'écoulement de liquide variable pouvant être contrôlé comprennent un dispositif à vide (40) qui est en communication fluidique avec le conduit de mesure (2) et qui est conçu pour établir une variation de différence de pression de part et d'autre du bouchon de particules pendant le cycle de mesure.

10. Système selon la revendication 9, dans lequel le dispositif à vide comprend un dispositif à cylindre et piston comportant un cylindre (42) et un piston pouvant se déplacer en va-et-vient (43) délimitant dans celui-ci une chambre à volume variable (44) en communication fluidique avec ledit conduit de mesure (2), dans lequel un moteur d'entraînement (45) associé est prévu pour effectuer un déplacement contrôlé dudit piston par rapport au dit cylindre, et dans lequel, de préférence, le dispositif à vide est conçu pour provoquer une variation périodique prédéterminée de la pression dans ladite chambre à volume variable (44).

11. Système selon une ou plusieurs des revendications précédentes, dans lequel ledit conduit de mesure (2) est agencé dans une orientation verticale, lesdites première et deuxième électrodes (4, 6) étant agencées l'une au-dessus de l'autre.

12. Système selon la revendication 10, comprenant en outre un capteur de pression qui permet la mesure de la pression dans ladite chambre à volume variable (44), et des moyens de commande (10) associés aux moyens d'entraînement (45), lesdits moyens de commande étant conçus pour établir un motif prédéterminé de variation de la pression dans ladite chambre à volume variable (44).

13. Système selon une ou plusieurs des revendications précédentes, dans lequel ledit système comprend en outre un capteur de pression conçu pour mesurer la différence de pression de part et d'autre du bouchon de particules, ledit capteur de pression étant interconnecté avec lesdits moyens de traitement de signal.

14. Système selon une ou plusieurs des revendications précédentes, dans lequel le système comprend un capteur de conductivité conçu pour mesurer la conductivité du liquide, ledit capteur de conductivité étant interconnecté avec lesdits moyens de traitement de signal.

15. Procédé pour la détermination d'une caractéristique électrique, en particulier du potentiel électrocinétique ou du potentiel zêta, d'un liquide, ledit procédé comprenant l'utilisation d'un système selon une ou plusieurs des revendications précédentes.
